# EUROPÄISCHE PATENTSCHRIFT

(11) **EP 4 350 177 B1**
(45) Veröffentlichungstag und Bekanntmachung des Hinweises auf die Patenterteilung: **29.10.2025**
(21) Anmeldenummer: 23195107.0
(22) Anmeldetag: 04.09.2023
(51) Int. Cl.: F16J 3/04, A61M 39/20, F16J 15/52

(54) **HYGIENEGERECHTES DICHTELEMENT**
HYGIENIC SEALING ELEMENT
ÉLÉMENT D'ÉTANCHÉITÉ HYGIÉNIQUE

(30) Priorität: 07.10.2022 DE 202022105676 U
(43) Veröffentlichungstag der Anmeldung: 10.04.2024
(73) Patentinhaber: Multipond Wägetechnik GmbH, 84478 Waldkraiburg (DE)
(72) Erfinder: PETERS, Andreas, 84544 Aschau am Inn (DE)
(74) Vertreter: Prüfer & Partner mbB Patentanwälte · Rechtsanwälte

(56) Entgegenhaltungen:
- DE-A1- 19 509 491
- DE-U- 1 970 306
- JP-Y1- S 485 052
- US-A1- 2012 248 712

## Beschreibung

Die vorliegende Anmeldung betrifft ein hygienegerecht ausgestaltetes Dichtelement, welches beispielsweise als Faltenbalg ausgeführt sein kann, sowie ein System zur Aufnahme des Dichtelements.

Ganz allgemein ist die Abdichtung beweglicher Bauteile an Gehäuseschnittstellen technisch anspruchsvoll, insbesondere wenn eine erhöhte Dichtigkeit verlangt wird, da die Abdichtung immer wieder Bewegungen unterzogen ist wodurch Hohlräume, Spalte usw. entstehen können. Eine solche dynamische Dichtung hat meistens das Ziel, zwei Bereiche vollständig voneinander abzugrenzen, um einen Transfer von Stoffen (wie beispielsweise Gasen, Flüssigkeiten) zwischen diesen Bereichen zu verhindern.

Im Stand der Technik sind hierzu Faltenbälge zur Verbindung von sich gegeneinander bewegenden Bauteilen bekannt. Faltenbälge haben den Vorteil, dass an der Schnittstelle eine Relativbewegung der kompletten Dichtung zu den sich bewegenden Bauteilen vermieden werden kann. Die Dichtung ist mit beiden Bauteilen verbunden, d.h. mit dem festen Bauteil und dem sich bewegenden Bauteil. Eine Relativbewegung wird durch die Dichtung selbst größtenteils ausgeglichen.

Ein Faltenbalg besteht in der Regel aus dünnem, elastischem Material, beispielsweise einer Elastomermembran. Eine solche Lösung bietet für die Bewegung eine Materialreserve und eine Bewegung des Faltenbalgs selbst sowie dessen Schnittstellen zu den abzudichtenden Bauteilen ist in jede Raumrichtung möglich.

Die Schnittstellen zu den angrenzenden Bauteilen, wie beispielsweise dem festen Bauteil und dem beweglichen Bauteil, wird häufig über Federringe, Schellen oder einen elastischen Bund an Bauteilen vorgenommen. Dies kann allerdings zu einer ungleichmäßigen oder undefinierten Verpressung am Bauteil führen, was wiederum zu einer Schädigung des Materials führen kann. Dadurch kann die Dichtigkeit des Faltenbalgs über die Zeit abnehmen.

Im Stand der Technik ist eine Lösung bekannt, in welcher eine formschlüssige Anbindung an das feste Bauteil und an das bewegliche Bauteil vorgenommen wird. Dokument KR102100807 B1 offenbart eine Luftfeder mit einem eingebauten Dämpfer, um eine wirksame Staubabdichtung einer Maschine mit einer sehr einfachen Konfiguration zu erreichen. Zur Befestigung der Luftfeder wird ein Fixierring verwendet.

Dokument EP 3 246 605 A1 offenbart ein Membranventil, bei welchem eine Membran entsprechend verklemmt wird.

Dokument DE1970306 U offenbart eine Rollmembrane als Dichtungselement zwischen Kolben und Zylinder.

Dokument DE 19509491 A1 offenbart ein System gemäß dem Oberbegriff des Anspruchs 1, und offenbart eine Rollmembran aus zumindest einem abdichtenden Material zur Anbringung zwischen einem zylinderförmigen Körper und einem den zylinderförmigen Körper umgebenden hohlzylinderförmigen Körper, zwischen welchen eine Relativbewegung in einer Achse erfolgt.

Dokument JPS485052_Y1 offenbart ein Dichtelement.

Dokument US20120248712 A1 offenbart eine Manschettendichtung für Motoren mit variabler Verdichtung.

Die Bauteile werden an den Schnittstellen mit angepasster und aufeinander abgestimmter Bauteilgeometrie mit definiertem Anschlag verbunden. Material des Faltenbalgs wird so definiert verpresst, so dass eine dauerhafte Materialschädigung somit verhindert werden kann.

Hier kann allerdings ein Nachteil entstehen, dass durch den Verbau von einem Klemmring oder einem Federring eine hygienische, gut zu reinigende Ausführungsform nicht möglich ist, da der Übergang vom Klemmring zum Faltenbalg nicht definiert abgedichtet ist, zwischen Klemmring und Faltenbalg ein nichtreinigbarer Zwischenraum entsteht (ein Klemmring oder ein Federring hat selbst eine sehr große Oberfläche), und die Verschraubung eines Klemmrings kann offenliegende Gewindeabschnitte enthalten.

Dadurch können Anlagenteile geschädigt werden, beispielsweise dadurch, dass Flüssigkeiten, Gas oder Schmutz oder Ölreste eindringen. Ferner ist eine Verunreinigung von Produkt, Anlagenteilen oder Umwelt möglich. Auch sich lösende Dichtungsbestandteile, was durch Bauteilschädigung entstehen kann, sind ein deutlicher Nachteil, da sie Produkte verunreinigen können. Ferner können umliegende Anlagenteile durch sich lösende Dichtungsbestandteile geschädigt werden, was einen erhöhten Reinigungs- und Wartungsaufwand und somit auch höhere Kosten nach sich ziehen kann. Kosten werden durch die Beschädigung von Bauteilen sehr schwer kalkulierbar, da Bauteilschädigungen auch zu längeren Anlagenstillständen führen können.

Es ist daher Aufgabe der vorliegenden Erfindung, eine dauerhafte, dichte und hygienegerechte Verbindung zwischen einem Dichtelement, wie beispielsweise einem Faltenbalg, sowie festen bzw. beweglichen Bauteilen bereitzustellen.

Diese Aufgabe wird gelöst durch ein System gemäß Anspruch 1. Weitere vorteilhafte Ausführungsformen der vorliegenden Erfindung sind Gegenstand der Unteransprüche.

Das erfindungsgemäße System umfasst:
eine Dichtvorrichtung mit einem Hauptkörper, an dem eine Verdickung vorgesehen ist, welche dazu angepasst ist, in eine Aufnahmevorrichtung eingesetzt zu werden,
einen Grundkörper sowie eine Klemmvorrichtung,
wobei die Klemmvorrichtung mit dem Grundkörper verbindbar ist, wobei im Grundkörper und/oder der Klemmvorrichtung eine Aufnahmevorrichtung vorgesehen ist, welche dazu angepasst ist, die Verdickung der Dichtvorrichtung aufzunehmen. Besagte Verdickungen sind Materialanhäufungen.

Die Verdickungen sind dazu angepasst, in jeweils eine erste Aufnahmevorrichtung und eine zweite Aufnahmevorrichtung eingesetzt zu werden.

Die Verdickungen sind in einer radial ausbauchenden Form ausgestaltet, und eine solche Ausführungsform ist für die Abdichtung von Schnittstellen gegen das Eintreten und Austreten von Stoffen besonders gut geeignet, da die Verdickungen eine sehr gute Dichtwirkung erzeugen. Ferner lassen es die Verdickungen zu, dass diese gut von Aufnahmevorrichtungen von Grundkörpern gehalten werden können, ohne dass hier entsprechende Spannungsspitzen entstehen können.

Dadurch ist ein definierter, spaltfreier Übergang zwischen Dichtvorrichtung und Grundkörpern, welche ja die Dichtvorrichtung befestigen sollen, vorhanden. Ferner können somit nicht-reinigbare Spalte den Übergang von der Dichtvorrichtung zum Grundkörper bzw. der Klemmvorrichtung vermieden werden. Ferner ist eine hygienische und sicher reinigbare Befestigung der Dichtvorrichtung vorhanden. Dadurch, dass ein Grundkörper und eine Klemmvorrichtung die Verdickung klemmt, ist eine definierte Verpressung der Schnittstellen, d.h. der Verdickung der Dichtvorrichtung vorhanden. Eine gute Dichtwirkung ist somit vorhanden und diese erfüllt Schutzklassen bis zu IP69.

Vorzugsweise sind an dem Hauptkörper an entsprechenden Enden jeweils eine erste und zweite Verdickung angeordnet, welche jeweils dazu angepasst sind, in jeweils eine erste Aufnahmevorrichtung und eine zweite Aufnahmevorrichtung eingesetzt zu werden.

Das System umfasst dann einen ersten Grundkörper, eine erste Klemmvorrichtung, einen zweiten Grundkörper und eine zweite Klemmvorrichtung.

Mit dem ersten Grundkörper ist eine erste Klemmvorrichtung verbindbar, und im ersten Grundkörper und/oder der ersten Klemmvorrichtung ist eine erste Aufnahmevorrichtung vorgesehen, welche dazu angepasst ist, die erste Verdickung der Dichtvorrichtung aufzunehmen, und/oder im zweiten Grundkörper und/oder der zweiten Klemmvorrichtung ist eine zweite Aufnahmevorrichtung vorgesehen, welche dazu angepasst ist, die zweite Verdickung der Dichtvorrichtung aufzunehmen.

Mit dem ersten Grundkörper ist somit eine erste Klemmvorrichtung verbindbar, und im ersten Grundkörper und/oder der ersten Klemmvorrichtung ist eine erste Aufnahmevorrichtung vorgesehen, welche dazu angepasst ist, die erste Verdickung in der Dichtvorrichtung aufzunehmen. Zusätzlich oder alternativ ist mit dem zweiten Grundkörper eine zweite Klemmvorrichtung verbindbar, und im zweiten Grundkörper und/oder der zweiten Klemmvorrichtung ist eine zweite Aufnahmevorrichtung vorgesehen, welche dazu angepasst ist, die zweite Verdickung der Dichtvorrichtung aufzunehmen. Die beiden Verdickungen werden somit zwischen erstem Grundkörper und erster Klemmvorrichtung bzw. zweitem Grundkörper und zweiter Klemmvorrichtung geklemmt.

Vorzugsweise ist der Hauptkörper als Faltenbalg ausgestaltet. Dieser kann eine oder mehrere Falten aufweisen. Hier ist eine Materialreserve vorhanden, wenn sich der erste Grundkörper und der zweite Grundkörper relativ zueinander bewegen, da sich die mindestens eine Falte entsprechend auffalten bzw. aufdehnen und wieder zusammenklappen bzw. zusammenziehen kann. Ein Faltenbalg behindert die Bewegung eines beweglichen Teils nur minimal. Ein Kraftnebenschluss kann so minimiert werden.

Der Hauptkörper und die erste und zweite Verdickung sind vorzugsweise aus elastischem Material, weiter vorzugsweise Elastomer ausgebildet. Solche Werkstoffe erlauben eine hohe Dauerfestigkeit und ferner eine hohe Elastizität.

Vorzugsweise sind der Hauptkörper sowie die erste und zweite Verdickung einstückig, also integral ausgebildet. Eine solche Dichtvorrichtung ist besonders einfach zu fertigen, und ferner entstehen keinerlei Eigenspannungen durch das Verwenden verschiedener Materialien.

Vorzugsweise ist der Übergang zwischen dem Hauptkörper sowie der ersten und zweiten Verdickung stufenlos ausgebildet. Hier ist ein stetiger Übergang zwischen dem Hauptkörper sowie der ersten und zweiten Verdickung, ohne Kanten, Stufen, Ausnehmungen usw.

Hier entstehen keine zusätzlichen Oberflächen, womit eine Reinigbarkeit und somit eine bessere hygienegerechte Gestaltung möglich ist. Dies ist insbesondere für die Verwendung der Lebensmittelindustrie oder lebensmittelverarbeitenden Industrie wichtig. Ferner ist so eine bessere Lastspitzenverteilung und dadurch eine erhöhte Dauerfestigkeit vorhanden.

Vorzugsweise ist die erste Aufnahmevorrichtung im radial äußeren Bereich des ersten Grundkörpers und der ersten Klemmvorrichtung vorgesehen ist, und/oder die zweite Aufnahmevorrichtung ist im radial äußeren Bereich des zweiten Grundkörpers und der zweiten Klemmvorrichtung vorgesehen. Dies sind Bereiche nahe der Außenkante der beiden Grundkörper bzw. Klemmvorrichtungen, also beispielsweise ca. 3 - 5 mm entfernt von der Außenkante.

Vorzugsweise ist die erste Aufnahmevorrichtung dazu angepasst, die erste Verdickung der Dichtvorrichtung zu fixieren, und die zweite Aufnahmevorrichtung ist dazu angepasst, die zweite Verdickung der Dichtvorrichtung zu fixieren. So kann die Dichtvorrichtung jeweils zwischen zwei Bauteilen geklemmt werden, denn die erste Aufnahmevorrichtung kann am ersten Grundkörper und der ersten Klemmvorrichtung vorgesehen sein, und die zweite Aufnahmevorrichtung kann an dem zweiten Grundkörper und/oder der zweiten Klemmvorrichtung vorgesehen sein.

Die erste Aufnahmevorrichtung ist eine Nut, welche am ersten Grundkörper und/oder der ersten Klemmvorrichtung vorgesehen ist. Die zweite Aufnahmevorrichtung ist ebenfalls eine Nut, welche im zweiten Grundkörper und/oder der zweiten Klemmvorrichtung vorgesehen ist.

Genauer gesagt umfasst die erste Aufnahmevorrichtung eine erste Nut im ersten Grundkörper und eine zweite Nut in der ersten Klemmvorrichtung. Die zweite Aufnahmevorrichtung umfasst dementsprechende eine dritte Nut im zweiten Grundkörper und eine vierte Nut in der zweiten Klemmvorrichtung.

Eine solche Ausführungsform stellt einen mechanischen Anschlag zur Verfügung (da die Nuten eine vordefinierte Größe aufweisen), damit kann eine definierte Verpressung der Verdickungen erreicht werden, welche eine sehr gute Dichtwirkung aufweist. Die Verdickungen des Dichtelements unterliegen vorzugsweise einer elastischen Verformung, nicht allerdings einer plastischen Verformung. Somit ist eine sehr gute Montagefreundlichkeit gegeben. Dies führt insbesondere dazu, dass ein definierter, spaltfreier Übergang geschaffen wird, die Befestigung des Dichtelements ist so hygienegerecht ausgestaltet, und ein leicht reinigbarer Übergang von Dichtelement zum ersten Grundkörper bzw. zweiten Grundkörper ist sichergestellt. Dadurch wird eine einfache und sichere Reinigung ermöglicht.

Vorzugsweise weisen die erste Nut, die zweite Nut, die dritte Nut und/oder die vierte Nut einen im Wesentlichen halbkreisförmigen, halbelliptischen oder polygonalen Querschnitt auf.

Die Nut ist vorzugsweise den Konturen der Verdickungen entsprechend angepasst - daher eignen sich hier beispielsweise runde Profile, es können aber auch polygonale oder halbelliptische Profile vorhanden sein. Jeweils zwei Halbschalten nehmen eine Verdickung auf.

Die erste Nut und die Außenkante des ersten Grundkörpers,
die zweite Nut und die Außenkante der ersten Klemmvorrichtung, die dritte Nut und die Außenkante des zweiten Grundkörpers und/oder die vierte Nut und die Außenkante der zweiten Klemmvorrichtung weisen jeweils eine spitz zulaufende Kante auf (diese hat einen spitzen Winkel, beispielsweise zwischen 10 und 45°. Diese hält die erste bzw. zweite Verdickung, auch bei Zugbeanspruchung, in den jeweiligen Nuten fest.

Vorzugsweise sind der erste Grundkörper und die erste Klemmvorrichtung durch eine erste Verbindungsvorrichtung verbindbar, und der zweite Grundkörper und die zweite Klemmvorrichtung sind durch eine zweite Verbindungsvorrichtung verbindbar. Die erste Verbindungsvorrichtung und/oder die zweite Verbindungsvorrichtung können als Gewindepaar, Schraube, Clip oder Bajonettverschluss ausgeführt sein.

In einer vorteilhaften Ausführungsform ist die erste Verbindungsvorrichtung eine Schraube, mit welcher der erste Grundkörper und die erste Klemmvorrichtung miteinander verbindbar sind, und die zweite Verbindungsvorrichtung ist ein Gewindepaar, weiter vorzugsweise ein Innengewinde, welches am zweiten Grundkörper vorgesehen ist, und ein Außengewinde, welches an der zweiten Klemmvorrichtung vorgesehen ist, umfasst.

Die Verpressung der ersten Verdickung des Dichtelements kann so nur durch das Verdrehen einer Schraube bzw. der ersten Klemmvorrichtung und der zweiten Klemmvorrichtung vorgenommen werden, dies ermöglicht eine gute Kraftverteilung und eine definierte Verpressung der Verdickungen, was wiederum zu einer sehr guten Dichtwirkung führt.

Vorzugsweise sind der zweite Grundkörper und die zweite Klemmvorrichtung jeweils mit einer Ausnehmung versehen, in welcher der erste Grundkörper konzentrisch angeordnet ist.

Vorzugsweise ist der zweite Grundkörper gegenüber dem ersten Grundkörper beweglich.

Eine solche Ausführungsform findet beispielsweise Anwendung in Linearantrieben.

Vorzugsweise ist das beschriebene System ein Dosierrinnenantrieb, ein Verteiltellerantrieb und ein Beschickungsantrieb einer Mehrkopfwaage oder dient zur Krafteinleitung in Kraftsensoren. Bei solchen Waagen, welche in der lebensmittelverarbeitenden Industrie eingesetzt werden, ist eine hygienegerechte Gestaltung äußerst wichtig, und aus diesem Grund hier von besonderem Vorteil.

Vorzugsweise weist der Hauptkörper eine höhere Elastizität auf als die erste und/oder zweite Verdickung. Die Verdickungen müssen nur in den Aufnahmevorrichtungen verpresst werden (mit elastischer, aber nicht plastischer Verformung) und können in einigen Ausführungsformen niedrigere Elastizitätserfordernisse haben als der Hauptkörper (z.B. Faltenbalg),

Eine erfindungsgemäße Dichtvorrichtung umfasst: einen Hauptkörper, an dessen Ende jeweils eine erste und zweite Verdickung angeordnet sind, wobei der Hauptkörper eine höhere Elastizität aufweist als die erste und/oder zweite Verdickung.

Im Folgenden werden bevorzugte Ausführungsformen der vorliegenden Erfindung anhand der beiliegenden Figuren näher beschrieben.
- Fig. 1: zeigt eine beispielhafte Ausführungsform, in welcher eine Dichtvorrichtung zum Einsatz kommt.
- Fig. 2: zeigt eine erste Ausführungsform, in welcher eine erfindungsgemäße Dichtung erfolgt.
- Fig. 3: zeigt eine Detailansicht, in welcher eine erste Verdickung einer erfindungsgemäßen Dichtvorrichtung entsprechend zwischen einem ersten Grundkörper und einer ersten Klemmvorrichtung geklemmt sind (Fig. 3 a)), bzw. eine Detailansicht, in welcher eine zweite Verdickung einer erfindungsgemäßen Dichtvorrichtung entsprechend zwischen einem zweiten Grundkörper und einer zweiten Klemmvorrichtung geklemmt sind (Fig. 3 b)).
- Fig. 4: zeigt eine zweite Ausführungsform der vorliegenden Erfindung, hier ist insbesondere die Verbindung zwischen erstem Grundkörper und erster Klemmvorrichtung sowie zweitem Grundkörper und zweiter Klemmvorrichtung näher gezeigt.
- Fig. 5: zeigt eine dritte Ausführungsform der vorliegenden Erfindung. Fig. 5 a) zeigt eine Detailansicht der Verdickungen der Dichtvorrichtung; Fig. 5 b) zeigt, wie eine Verdickung einer erfindungsgemäßen Dichtvorrichtung entsprechend zwischen einem ersten Grundkörper und einer ersten Klemmvorrichtung geklemmt sind.
- Fig. 6: zeigt einen Faltenbalg mit einer Falte zur Verbindung radial versetzter Schnittstellen.
- Fig. 7: zeigt einen Faltenbalg mit mehreren Falten zur Minimierung des Kraftschlusses bzw. größeren relativen Bewegungen.
- Fig. 8: zeigt einen Faltenbalg mit einer Falte zur Verbindung radial versetzter Schnittstellen, wobei der Faltenbalg an beiden Grundkörpern innen befestigt ist.

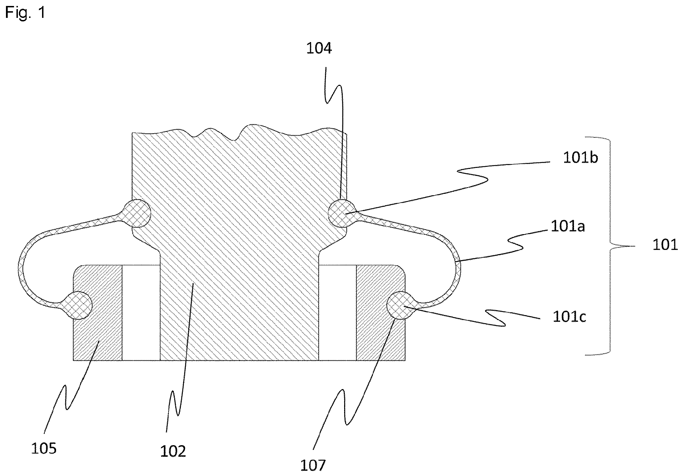

Fig. 1 zeigt eine beispielhafte Ausführungsform einer Befestigung einer Dichtvorrichtung 101 (mit einem Hauptkörper 101a, einer ersten Verdickung 101b und einer zweiten Verdickung 1c). Die erste Verdickung 101b ist in einer ersten Aufnahmevorrichtung 104 eines ersten Grundkörpers 102 vorgesehen, die zweite Verdickung 101b ist in einer zweiten Aufnahmevorrichtung 107 eines zweiten Grundkörpers 105 vorgesehen. Der erste Grundkörper 102 und der zweite Grundkörper 105 sind relativ zueinander beweglich. Die erste Verdickung 101b und zweite Verdickung 101c müssen stark aufgedehnt werden, um in die erste Aufnahmevorrichtung 104 und zweite Aufnahmevorrichtung 107 gezogen zu werden.

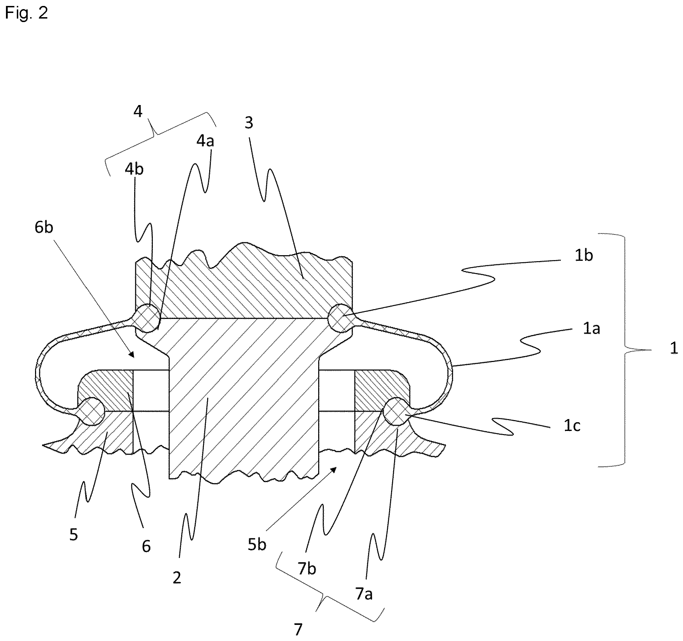

Fig. 2 zeigt das Grundprinzip der vorliegenden Anmeldung. Hier sind ein erster Grundkörper 2 sowie eine erste Klemmvorrichtung 3 vorhanden, welche miteinander verbunden sind. Diese sind in einer Ausnehmung 5b des zweiten Grundkörpers 5 angeordnet, der zweite Grundkörper 5 und der erste Grundkörper 2 sind konzentrisch zueinander. Auf dem zweiten Grundkörper 5 ist eine zweite Klemmvorrichtung 6 vorgesehen. Auch in dieser befindet sich eine entsprechende Ausnehmung 6b.

Der erste Grundkörper 2 ist relativ zum zweiten Grundkörper 3 beweglich.

In dem ersten Grundkörper 2 und der ersten Klemmvorrichtung 3 sind jeweils im radial äußeren Bereich eine erste Nut 4a und eine zweite Nut 4b vorgesehen, diese bilden zusammen die erste Aufnahmevorrichtung 4. In diese Aufnahmevorrichtung 4 ist eine erste Verdickung 1b der Dichtvorrichtung 1 geklemmt. In dem zweiten Grundkörper 5 bzw. der zweiten Klemmvorrichtung 6 sind jeweils eine dritte Nut 7a und eine vierte Nut 7b vorgesehen, diese bilden zusammen die zweite Aufnahmevorrichtung 7. Diese Nuten 7a und 7b klemmen zusammen die zweite Verdickung 1c der Dichtvorrichtung 1 fest. Zwischen dem ersten Grundkörper 2 und der ersten Klemmvorrichtung 3 sowie dem zweiten Grundkörper 5 und der zweiten Klemmvorrichtung 6 ist somit kein Spalt vorhanden, es entstehen keine Hohlräume, Rillen oder sonstige Vertiefungen, in welchen sich Schmutz, Reinigungsmittel, Lebensmittelreste, usw. ansammeln könnten - denn die Nuten 4a und 4b sind vollständig durch die erste Verdickung 1b ausgefüllt, und die Nuten 7a und 7b sind vollständig durch die zweite Verdickung 1c ausgefüllt. Die Ausnehmung 5b des zweiten Grundkörpers 5 sowie die Ausnehmung 6b der zweiten Klemmvorrichtung 6 sind nun gegenüber dem Außenraum hermetisch abgedichtet. Auch wenn außerhalb der Dichtvorrichtung 1 höherer Druck anliegen sollte, kann dieser durch die erste Verdickung 1b bzw. zweite Verdickung 1c, welche entsprechend geklemmt sind, nicht in das Innere eindringen, insbesondere nicht in die Ausnehmungen 5b bzw. 6b. Zwischen der ersten Verdickung 1b und der zweiten Verdickung 1c ist ein Hauptkörper 1a der Dichtvorrichtung vorgesehen. Dieser kann beispielsweise aus Faltenbalg ausgeführt sein.

Da die Übergänge zwischen erstem Grundkörper 2 und erster Klemmvorrichtung 3 und dem zweiten Grundkörper 5 sowie der zweiten Klemmvorrichtung 6 von der Dichtvorrichtung 1 abgedeckt sind, ist von außen kein Unterschied im Vergleich zu Fig. 1 erkennbar. Alle Unterschiede sind von der Dichtvorrichtung 1 abgedeckt.

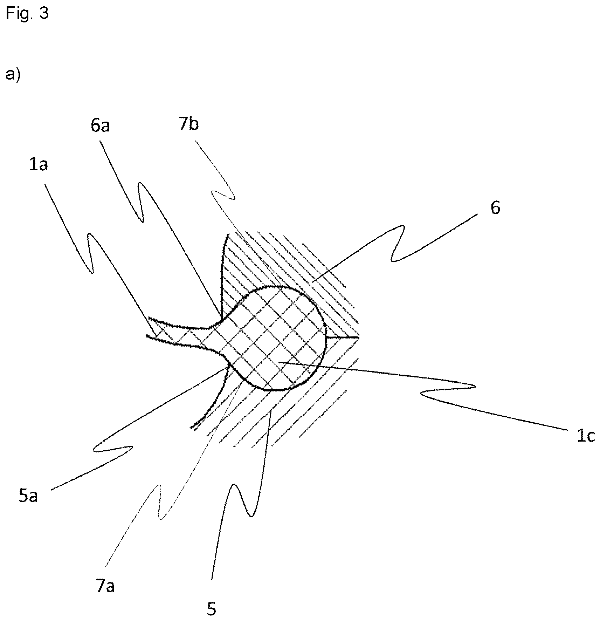

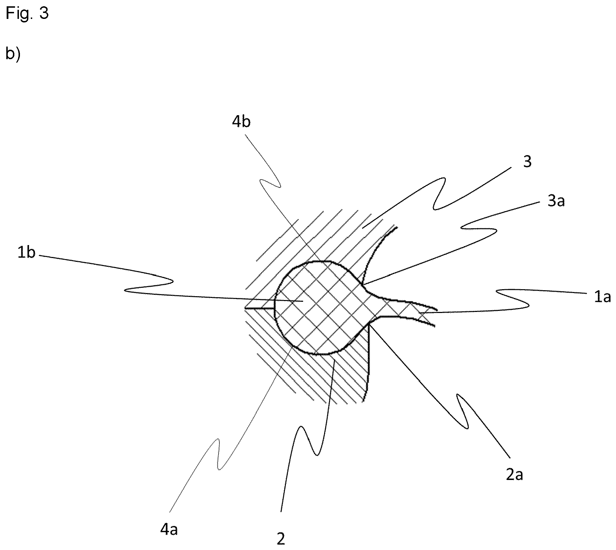

Fig. 3 a) zeigt eine Detailansicht einer zweiten Verdickung 1c, an welcher sich ein Hauptkörper 1a einer Dichtvorrichtung anschließt. Im zweiten Grundkörper 5 ist eine dritte Nut 7a mit Halbkreisprofil vorgesehen, entsprechend ist in der zweiten Klemmvorrichtung 6 eine vierte Nut 7b vorgesehen, auch diese hat ein Halbkreisprofil. Die Nuten 7a und 7b sind radial im äußeren Bereich des zweiten Grundkörpers 5 sowie der zweiten Klemmvorrichtung 6 angeordnet. Am radial äußeren Ende des zweiten Grundkörpers 5 und der zweiten Klemmvorrichtung 6 ist jeweils eine dritte Kante 5a bzw. eine vierte Kante 6a vorgesehen. Diese Kanten 5a, 6a laufen spitz zu und klemmen somit die zweite Verdickung 1c ein und erlauben keinen Spalt zwischen zweitem Grundkörper 5 bzw. zweiten Klemmvorrichtung 6 und zweite Verdickung 1c. Wenn Zug bzw. Spannung am Hauptkörper 1a auftritt, stellen diese dritte Kante 5a und vierte Kante 6a auch sicher, dass die erste Verdickung sicher in den Nuten 7a und 7b gehalten wird und nicht herausgezogen werden kann - gleichzeitig, dass keine Schäden bzw. Risse in Hauptkörper 1a oder zweiter Verdickung 1c auftreten. Dies ist insbesondere von Vorteil, wenn die Dichtvorrichtung 1 häufig Bewegungen unterzogen wird.

Fig. 3 b) zeigt eine Detailansicht einer ersten Verdickung 1b, an welcher sich ein Hauptkörper 1a einer Dichtvorrichtung anschließt. Im ersten Grundkörper 2 ist eine Nut 4a mit Halbkreisprofil vorgesehen, entsprechend ist in der ersten Klemmvorrichtung 3 eine zweite Nut 4b vorgesehen, auch diese hat ein Halbkreisprofil. Die Nuten 4a und 4b sind radial im äußeren Bereich des ersten Grundkörpers 2 sowie der ersten Klemmvorrichtung 3 angeordnet. Am radial äußeren Ende des ersten Grundkörpers 2 und der ersten Klemmvorrichtung 3 ist jeweils eine erste Kante 2a bzw. eine zweite Kante 3a vorgesehen. Diese Kanten 2a, 3a laufen spitz zu und klemmen somit die erste Verdickung 1b ein und erlauben keinen Spalt zwischen erstem Grundkörper 2 bzw. erster Klemmvorrichtung 3 und erster Verdickung 1b. Wenn Zug bzw. Spannung am Hauptkörper 1a auftritt, stellen diese erste Kante 2a und zweite Kante 3a auch sicher, dass die erste Verdickung sicher in den Nuten 4a und 4b gehalten wird und nicht herausgezogen werden kann - gleichzeitig, dass keine Schäden bzw. Risse in Hauptkörper 1a oder erster Verdickung 1b auftreten. Dies ist insbesondere von Vorteil, wenn die Dichtvorrichtung 1 häufig Bewegungen unterzogen wird.

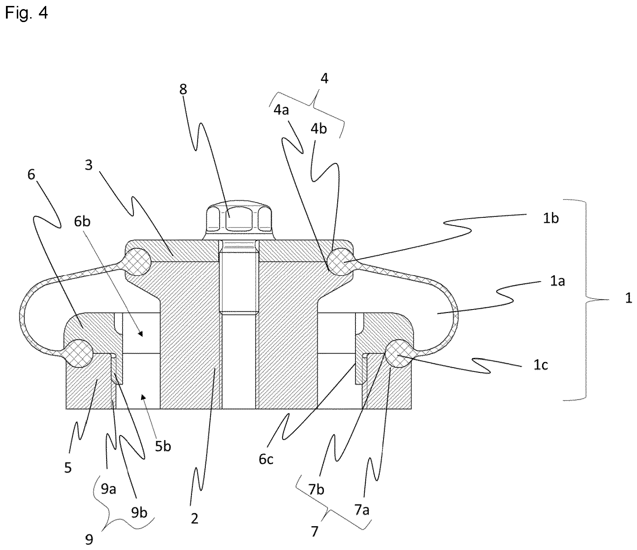

In Fig. 4 ist eine zweite Ausführungsform beschrieben, hier ist eine Dichtvorrichtung 1, bestehend aus Hauptkörper 1a, erster Verdickung 1b und zweiter Verdickung 1c, wiederum zwischen einem ersten Grundkörper 2 und einem zweiten Grundkörper 5 vorgesehen. Der erste Grundkörper 2 wird wiederum von einer ersten Klemmvorrichtung 3 bedeckt, hier sind eine erste Nut 4a und eine zweite Nut 4b vorgesehen, welche zusammen die erste Aufnahmevorrichtung 4 bilden, in welcher die erste Verdickung 1b geklemmt wird.

Die Verbindung zwischen erstem Grundkörper 2 und erster Klemmvorrichtung 3 erfolgt in diesem Fall durch eine erste Verbindungsvorrichtung 8, welche in der vorliegenden Ausführungsform eine Schraube ist. Die zweite Verdickung 1c ist zwischen zweitem Grundkörper 5 und zweiter Klemmvorrichtung 6 vorgesehen, auch hier sind wiederum entsprechende Nuten 7a und 7b vorhanden, welche die zweite Aufnahmevorrichtung 7 ausbilden. Innerhalb des zweiten Grundkörpers ist eine Ausnehmung 5b vorhanden, entsprechend ist auch in der zweiten Klemmvorrichtung 6 eine Ausnehmung 6b vorgesehen. Innerhalb dieser Ausnehmung ist der erste Grundkörper 2 konzentrisch angeordnet. Der zweite Grundkörper 5 und die zweite Klemmvorrichtung 6 sind mit Hilfe einer zweiten Verbindungsvorrichtung 9 verbunden, diese besteht aus einem ersten Gewinde 9a und einem zweiten Gewinde 9b. An der Unterseite der zweiten Klemmvorrichtung ist ein Vorsprung 6c vorgesehen, in diesem ist das zweite Gewinde 9b als Außengewinde vorgesehen. Das erste Gewinde 9a ist als ein Innengewinde innerhalb der Ausnehmung 5b des zweiten Grundkörpers 5 vorgesehen. Somit kann die zweite Klemmvorrichtung 6 in den zweiten Grundkörper 5 eingeschraubt werden, so dass die zweite Verdickung 1c der Dichtvorrichtung 1 sicher in der zweiten Aufnahmevorrichtung 7, welche aus einer dritten Nut 7a und vierten Nut 7b besteht, geklemmt werden. Es erfolgt so eine radial verteilte Verpressung der zweiten Verdickung 1c innerhalb der zweiten Aufnahmevorrichtung 7.

Somit kann die zweite Verdickung 1c zuerst geklemmt werden, da die in den zweiten Grundkörper 5 einschraubbare zweite Klemmvorrichtung 6 gut zugänglich ist. Anschließend kann die erste Verdickung 1b entsprechend zwischen dem ersten Grundkörper 2 und der ersten Klemmvorrichtung 3 geklemmt werden. Die Schraube 8 ist von außen dann gut zugänglich. Der erste Grundkörper 2 ist wiederum relativ zum zweiten Grundkörper 3 beweglich.

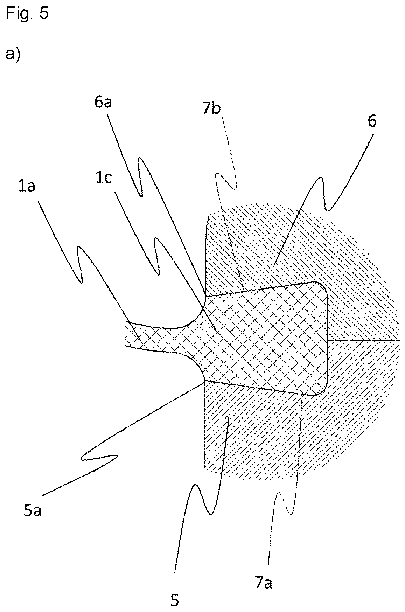

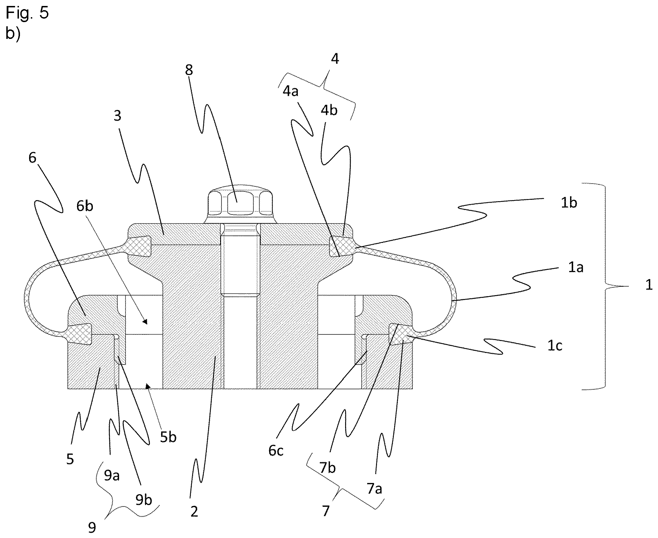

Fig. 5 a) zeigt eine ähnliche Darstellung wie die Fig. 2. Die zweite Verdickung 1c ist hier allerdings trapezförmig ausgebildet. Die Form der dritten Nut 7a im zweiten Grundkörper 5 bzw. der vierten Nut 7b in der zweiten Klemmvorrichtung 6 ist entsprechend angepasst. Die dritte Kante 5a und die vierte Kante 6a weisen einen spitzen Winkel auf - dieser ist jedoch größer (d.h. weniger spitz) wie in Fig. 3. Die erste Verdickung 1b, die erste Nut 4a und die zweite Nut 4b sind nicht dargestellt - diese haben aber dieselben Formen die die zweite Verdickung 1c, die dritte Nut 7a und vierte Nut 7b.

Fig. 5 b) zeigt den Einsatz der in Fig. 5 a) gezeigten zweiten Verdickung 1c. Bis auf die Form der ersten Verdickung 1b, zweiten Verdickung 1c, erster Nut 4a, zweiter Nut 4b, dritter Nut 7a und vierter Nut 7b sind alle Komponenten des Systems gleich wie in Fig. 4.

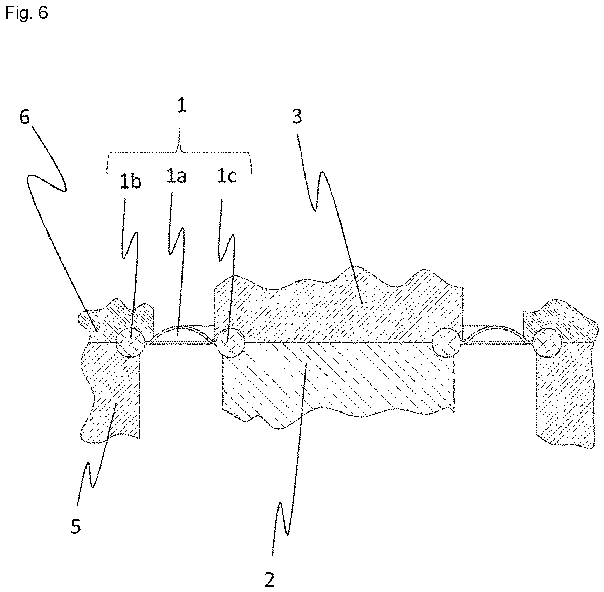

Fig. 6 zeigt eine Dichtvorrichtung 1 (wiederum mit Hauptkörper 1a, erster Verdickung 1b sowie zweiter Verdickung 1c) als Faltenbalg zur Verbindung radial versetzter Schnittstellen, auch hier kann mit Hilfe des neuartigen Verbindungskonzepts eine gute Abdichtung und eine gute Dauerfestigkeit erreicht werden. Der erste Grundkörper 2 und die erste Klemmvorrichtung 3 sind radial innen, der zweite Grundkörper 5 sowie die zweite Klemmvorrichtung 6 sind radial außen angeordnet. Schlecht reinigbare Bereiche können so vermieden werden.

Die Dichtvorrichtung verbindet eine Außenseite des ersten Grundkörpers 2 und der ersten Klemmvorrichtung 3 mit einer Innenseite des zweiten Grundkörpers 5 sowie die zweiten Klemmvorrichtung 6.

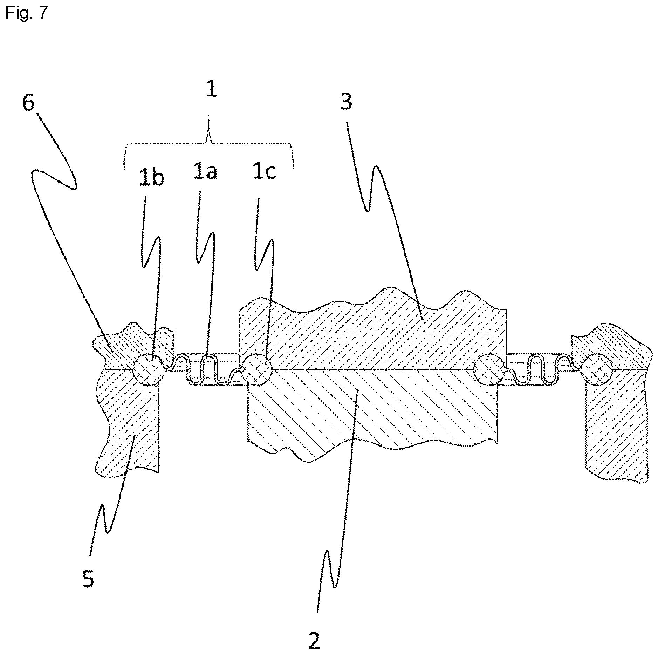

Fig. 7 zeigt ebenfalls eine Dichtvorrichtung 1 zur Verbindung radial versetzter Schnittstellen, im Unterscheid zu Fig. 6 allerdings mit mehreren Falten im Hauptkörper 1, zur Minimierung des Kraftnebenschlusses bzw. für größere relative Bewegungen.

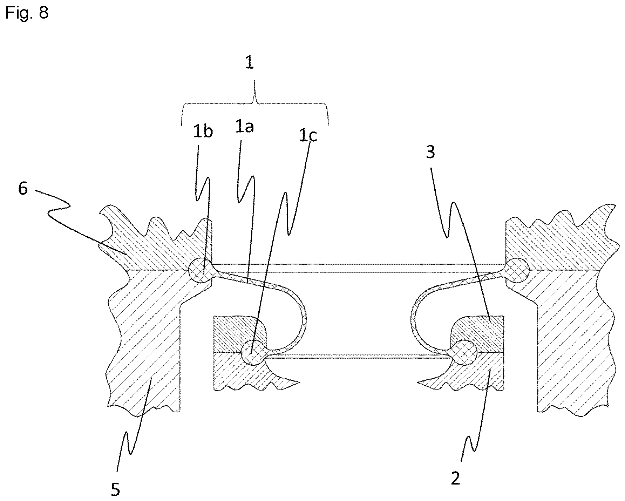

Fig. 8 zeigt ebenfalls eine Dichtvorrichtung 1 zur Verbindung radial versetzter Schnittstellen. Im Unterschied zu Fig. 6 verbindet die Dichtvorrichtung 1 allerdings eine Innenseite des ersten Grundkörpers 2 und der ersten Klemmvorrichtung 3 mit einer Innenseite des zweiten Grundkörpers 5 sowie die zweiten Klemmvorrichtung 6.

Die vorliegende Erfindung ist nicht auf die oben genannten Ausführungsformen beschränkt. Beispielsweise können die erste Verdickung 1b und die zweite Verdickung 1c verschiedene Formen aufweisen - es ist lediglich wichtig, dass im Vergleich zum Hauptkörper 1a der Dichtvorrichtung 1 eine Materialanhäufung vorhanden ist. Zudem kann die Verbindung von erstem Grundkörper 2 und erstem Klemmelement 3 bzw. zweitem Grundkörper 5 und zweitem Klemmelement 6 auch durch unlösbar vorgesehen sein, z.B. durch Kleben, Schweißen usw. Der erste Grundkörper 2, der zweite Grundkörper 5, die erste Klemmvorrichtung 3 sowie die zweite Klemmvorrichtung 6 können aus Metall (z.B. Edelstahl, Aluminium) oder Kunststoff ausgebildet sein.

### BEZUGSZEICHENLISTE

- 1: Dichtvorrichtung / Faltenbalg
- 1a: Hauptkörper
- 1b: erste Verdickung
- 1c: zweite Verdickung
- 2: erster Grundkörper
- 2a: erste Kante
- 3: erste Klemmvorrichtung
- 3a: zweite Kante
- 4: erste Aufnahmevorrichtung
- 4a: erste Nut
- 4b: zweite Nut
- 5: zweiter Grundkörper
- 5a: dritte Kante
- 5b: Ausnehmung des zweiten Grundkörpers
- 6: zweite Klemmvorrichtung
- 6a: vierte Kante
- 6b: Ausnehmung der zweiten Klemmvorrichtung
- 6c: Vorsprung
- 7: zweite Aufnahmevorrichtung
- 7a: dritte Nut
- 7b: vierte Nut
- 8: erste Verbindungsvorrichtung
- 9: zweite Verbindungsvorrichtung
- 9a: erstes Gewinde
- 9b: zweites Gewinde

- 101: Dichtvorrichtung
- 101a: Hauptkörper
- 101b: erste Verdickung
- 101c: zweite Verdickung
- 102: erster Grundkörper
- 104: erste Aufnahmevorrichtung
- 105: zweiter Grundkörper
- 107: zweite Aufnahmevorrichtung

## Patentansprüche

1. System, umfassend:
eine Dichtvorrichtung (1) mit einem Hauptkörper (1a), an dem eine Verdickung (1b, 1c) vorgesehen ist, welche dazu angepasst ist, in eine erste bzw. zweite Aufnahmevorrichtung (4, 7) eingesetzt zu werden,
einen ersten Grundkörper (2) sowie einen zweiten Grundkörper (5) sowie eine erste Klemmvorrichtung (3) und zweite Klemmvorrichtung (6),
wobei die erste Klemmvorrichtung (3) mit dem ersten Grundkörper (2) sowie die zweite Klemmvorrichtung (6) mit dem zweiten Grundkörper (5) verbindbar ist,
wobei im ersten und zweiten Grundkörper (2, 5) und/oder der ersten und zweiten Klemmvorrichtung (3, 6) eine Aufnahmevorrichtung (4, 4a, 4b; 7, 7a, 7b) vorgesehen ist, welche dazu angepasst ist, die Verdickung (1b, 1c) der Dichtvorrichtung (1) aufzunehmen, wobei eine erste Aufnahmevorrichtung (4) eine erste Nut (4a), welche im ersten Grundkörper (2) vorgesehen ist, und eine zweite Nut (4b), welche in der ersten Klemmvorrichtung (3) vorgesehen ist, umfasst, und/oder wobei eine zweite Aufnahmevorrichtung (7) eine dritte Nut (7a), welche im zweiten Grundkörper (5) vorgesehen ist, und eine vierte Nut (7b), welche in der zweiten Klemmvorrichtung (6) vorgesehen ist, umfasst,
**dadurch gekennzeichnet, dass**
die erste Nut (4a) und eine Außenkante des ersten Grundkörpers (2), die zweite Nut (4b) und eine Außenkante der ersten Klemmvorrichtung (3), die dritte Nut (7a) und eine Außenkante des zweiten Grundkörpers (2) und/oder die vierte Nut (7b) und eine Außenkante der zweiten Klemmvorrichtung (6) eine spitz zulaufende Kante (2a, 3a, 5a, 6a), welche einen spitzen Winkel hat, aufweisen.

2. System gemäß Anspruch 1, wobei
an dem Hauptkörper (1a) an entsprechenden Enden jeweils eine erste und zweite Verdickung (1b, 1c) angeordnet sind, welche jeweils dazu angepasst sind, in jeweils die erste Aufnahmevorrichtung (4) und die zweite Aufnahmevorrichtung (7) eingesetzt zu werden,
wobei mit dem ersten Grundkörper (2) die erste Klemmvorrichtung (3) verbindbar ist, wobei im ersten Grundkörper (2) und/oder der ersten Klemmvorrichtung (3) eine die Aufnahmevorrichtung (4, 4a, 4b) vorgesehen ist, welche dazu angepasst ist, die erste Verdickung (1b) der Dichtvorrichtung (1) aufzunehmen, und/oder wobei im zweiten Grundkörper (5) und/oder der zweiten Klemmvorrichtung (6) die zweite Aufnahmevorrichtung (7, 7a, 7b) vorgesehen ist, welche dazu angepasst ist, die zweite Verdickung (1c) der Dichtvorrichtung (1) aufzunehmen.

3. System gemäß Anspruch 1 oder 2, wobei der Hauptkörper (1a) der Dichtvorrichtung (1) als Faltenbalg ausgestaltet ist.

4. System gemäß einem der vorhergehenden Ansprüche, wobei der Hauptkörper (1a) und/oder die erste und zweite Verdickung (1b, 1c) aus elastischem Material, vorzugsweise Elastomer ausgebildet sind.

5. System gemäß einem der vorhergehenden Ansprüche, wobei der Hauptkörper (1a) sowie die erste und zweite Verdickung (1b, 1c) einstückig ausgebildet sind.

6. System gemäß einem der vorhergehenden Ansprüche, wobei der Übergang zwischen dem Hauptkörper (1a) sowie der ersten und/oder zweiten Verdickung (1b, 1c) jeweils stufenlos ausgebildet ist.

7. System gemäß einem der vorhergehenden Ansprüche, wobei die erste Aufnahmevorrichtung (4, 4a, 4b) im radial äußeren Bereich des ersten Grundkörpers (2) und der ersten Klemmvorrichtung (3) vorgesehen ist, und/oder wobei die zweite Aufnahmevorrichtung (7, 7a, 7b) im radial äußeren Bereich des zweiten Grundkörpers (5) und der zweiten Klemmvorrichtung (6) vorgesehen ist, wobei vorzugsweise die erste Aufnahmevorrichtung (4, 4a, 4b) dazu angepasst ist, die erste Verdickung (1b) der Dichtvorrichtung (1) zu fixieren, und/oder wobei die zweite Aufnahmevorrichtung (7, 7a, 7b) dazu angepasst ist, die zweite Verdickung (1c) der Dichtvorrichtung (1) zu fixieren.

8. System gemäß einem der Ansprüche 1 bis 7, wobei die erste Nut (4a), die zweite Nut (4b), die dritte Nut (7a) und/oder die vierte Nut (7b) einen im Wesentlichen halbkreisförmigen, halbelliptischen oder polygonalen Querschnitt aufweisen.

9. System gemäß einem der vorhergehenden Ansprüche, wobei der erste Grundkörper (2) und die erste Klemmvorrichtung (3) durch eine erste Verbindungsvorrichtung (8) verbindbar sind, und/oder wobei der zweite Grundkörper (5) und die zweite Klemmvorrichtung (6) durch eine zweite Verbindungsvorrichtung (9) verbindbar sind.

10. System gemäß Anspruch 9, wobei die erste Verbindungsvorrichtung (8) und/oder die zweite Verbindungsvorrichtung (9) als Gewindepaar, Schraube, Bajonettverschluss oder Clip ausgeführt sind.

11. System gemäß Anspruch 9, wobei die erste Verbindungsvorrichtung (8) eine Schraube ist, mit welcher der erste Grundkörper (2) und die erste Klemmvorrichtung (3) miteinander verbindbar sind, und/oder wobei die zweite Verbindungsvorrichtung (9) ein Gewindepaar ist, welches ein Innengewinde (9a), welches am zweiten Grundkörper (5) vorgesehen ist, und ein Außengewinde (9b), welches an der zweiten Klemmvorrichtung (6) vorgesehen ist, umfasst.

12. System gemäß einem der vorhergehenden Ansprüche, wobei der zweite Grundkörper (5) und die zweite Klemmvorrichtung (6) jeweils eine Ausnehmung (5b, 6b) aufweisen, in welchen der erste Grundkörper (2) konzentrisch angeordnet ist.

13. System gemäß einem der vorhergehenden Ansprüche, wobei der zweite Grundkörper (5) gegenüber dem ersten Grundkörper (2) beweglich ist.

14. System gemäß Anspruch 13, welches ein Dosierrinnenantrieb, ein Verteiltellerantrieb, ein Beschickungsantrieb einer Mehrkopfwaage ist oder für die Krafteinleitung in einen Kraftsensor dient.

15. System gemäß einem der vorhergehenden Ansprüche, wobei der Hauptkörper (1a) eine höhere Elastizität aufweist als die erste und/oder zweite Verdickung (1b, 1c).

## Claims

1. A system comprising:
a sealing device (1) having a main body (1a) on which a thickening (1b, 1c) is provided which is adapted to be inserted into a first and second receiving device (4, 7), respectively,
a first main body (2) and a second main body (5) and a first clamping device (3) and second clamping device (6),
wherein the first clamping device (3) is connectable to the first main body (2) and the second clamping device (6) is connectable to the second main body (5), wherein a receiving device (4, 4a, 4b; 7, 7a, 7b) is provided in the first and second main body (2, 5) and/or the first and second clamping device (3, 6),
which receiving device is adapted to receive the thickening (1b, 1c) of the sealing device (1), wherein a first receiving device (4) comprises a first groove (4a) which is provided in the first main body (2) and a second groove (4b) which is provided in the first clamping device (3), and/or wherein a second receiving device (7) comprises a third groove (7a) which is provided in the second main body (5) and a fourth groove (7b) which is provided in the second clamping device (6),
**characterized in that** the first groove (4a) and an outer edge of the first main body (2), the second groove (4b) and an outer edge of the first clamping device (3), the third groove (7a) and an outer edge of the second main body (2) and/or the fourth groove (7b) and an outer edge of the second clamping device (6) have a tapered edge (2a, 3a, 5a, 6a) which has an acute angle.

2. The system according to claim 1, wherein
a first and second thickening (1b, 1c) are respectively arranged on the main body (1a) at corresponding ends, which thickenings are respectively adapted to be inserted into the first receiving device (4) and the second receiving device (7), respectively,
wherein the first clamping device (3) is connectable to the first main body (2), wherein a second receiving device (4, 4a, 4b) is provided in the first main body (2) and/or the first clamping device (3), which second receiving device is adapted to receive the first thickening (1b) of the sealing device (1), and/or
wherein the second receiving device (7, 7a, 7b) is provided in the second main body (5) and/or the second clamping device (6), which second receiving device is adapted to receive the second thickening (1c) of the sealing device (1).

3. The system according to claim 1 or 2, wherein the main body (1a) of the sealing device (1) is configured as a bellows.

4. The system according to any one of the preceding claims, wherein the main body (1a) and/or the first and second thickening (1b, 1c) are formed from elastic material, preferably elastomer.

5. The system according to any one of the preceding claims, wherein the main body (1a) and the first and second thickening (1b, 1c) are integrally formed.

6. The system according to any one of the preceding claims, wherein the transition between the main body (1a) and the first and/or second thickening (1b, 1c) is respectively formed continuously.

7. The system according to any one of the preceding claims, wherein the first receiving device (4, 4a, 4b) is provided in the radially outer region of the first main body (2) and the first clamping device (3), and/or wherein the second receiving device (7, 7a, 7b) is provided in the radially outer region of the second main body (5) and the second clamping device (6), wherein preferably the first receiving device (4, 4a, 4b) is adapted to fix the first thickening (1b) of the sealing device (1), and/or wherein the second receiving device (7, 7a, 7b) is adapted to fix the second thickening (1c) of the sealing device (1).

8. The system according to any one of claims 1 to 7, wherein the first groove (4a), the second groove (4b), the third groove (7a) and/or the fourth groove (7b) have a substantially semicircular, semi-elliptical or polygonal cross-section.

9. The system according to any one of the preceding claims, wherein the first main body (2) and the first clamping device (3) are connectable by a first connecting device (8), and/or wherein the second main body (5) and the second clamping device (6) are connectable by a second connecting device (9).

10. The system according to claim 9, wherein the first connecting device (8) and/or the second connecting device (9) are configured as a thread pair, screw, bayonet closure or clip.

11. The system according to claim 9, wherein the first connecting device (8) is a screw, by which the first main body (2) and the first clamping device (3) are connectable to each other, and/or wherein the second connecting device (9) is a thread pair, which comprises an internal thread (9a), which is provided on the second main body (5), and an external thread (9b), which is provided on the second clamping device (6).

12. The system according to any one of the preceding claims, wherein the second main body (5) and the second clamping device (6) respectively have a recess (5b, 6b), in which the first main body (2) is concentrically arranged.

13. The system according to any one of the preceding claims, wherein the second main body (5) is movable with respect to the first main body (2).

14. The system according to claim 13, which is a dosing chute drive, a distribution plate drive, a loading drive of a multihead scale, or serves for the force introduction into a force sensor.

15. The system according to any one of the preceding claims, wherein the main body (1a) has a higher elasticity than the first and/or second thickening (1b, 1c).

## Revendications

1. Système comprenant :
un dispositif d'étanchéité (1) avec un corps principal (1a) sur lequel est prévu un épaississement (1b, 1c) qui est adapté pour être inséré dans un premier ou un deuxième dispositif de réception (4, 7),
un premier corps de base (2) ainsi qu'un deuxième corps de base (5) ainsi qu'un premier dispositif de serrage (3) et un deuxième dispositif de serrage (6), le premier dispositif de serrage (3) pouvant être relié au premier corps de base (2) et le deuxième dispositif de serrage (6) pouvant être relié au deuxième corps de base (5), un dispositif de réception (4, 4a, 4b ; 7, 7a, 7b) étant prévu dans le premier et le deuxième corps de base (2, 5) et/ou dans le premier et le deuxième dispositif de serrage (3, 6), lequel est adapté pour recevoir l'épaississement (1b, 1c) du dispositif d'étanchéité (1), un premier dispositif de réception (4) comprenant une première rainure (4a) qui est prévue dans le premier corps de base (2) et une deuxième rainure (4b) qui est prévue dans le premier dispositif de serrage (3), et/ou un deuxième dispositif de réception (7) comprenant une troisième rainure (7a) qui est prévue dans le deuxième corps de base (5) et une quatrième rainure (7b) qui est prévue dans le deuxième dispositif de serrage (6),
**caractérisé en ce que** la première rainure (4a) et un bord extérieur du premier corps de base (2), la deuxième rainure (4b) et un bord extérieur du premier dispositif de serrage (3), la troisième rainure (7a) et un bord extérieur du deuxième corps de base (2) et/ou la quatrième rainure (7b) et un bord extérieur du deuxième dispositif de serrage (6) présentent un bord effilé (2a, 3a, 5a, 6a) qui présente un angle aigu.

2. Système selon la revendication 1,
un premier et un deuxième épaississement (1b, 1c) étant respectivement disposés sur le corps principal (1a) à des extrémités correspondantes, lesquels sont respectivement adaptés pour être insérés respectivement dans le premier dispositif de réception (4) et dans le deuxième dispositif de réception (7),
le premier dispositif de serrage (3) pouvant être relié au premier corps de base (2), un premier dispositif de réception (4, 4a, 4b) étant prévu dans le premier corps de base (2) et/ou dans le premier dispositif de serrage (3), lequel est adapté pour recevoir le premier épaississement (1b) du dispositif d'étanchéité (1), et/ou le deuxième dispositif de réception (7, 7a, 7b) étant prévu dans le deuxième corps de base (5) et/ou dans le deuxième dispositif de serrage (6), lequel est adapté pour recevoir le deuxième épaississement (1c) du dispositif d'étanchéité (1).

3. Système selon la revendication 1 ou 2, le corps principal (1a) du dispositif d'étanchéité (1) étant configuré sous forme de soufflet.

4. Système selon l'une quelconque des revendications précédentes, le corps principal (1a) et/ou le premier et le deuxième épaississement (1b, 1c) étant réalisés en matériau élastique, de préférence en élastomère.

5. Système selon l'une quelconque des revendications précédentes, le corps principal (1a) ainsi que le premier et le deuxième épaississement (1b, 1c) étant réalisés en monobloc.

6. Système selon l'une quelconque des revendications précédentes, la transition entre le corps principal (1a) ainsi que le premier et/ou le deuxième épaississement (1b, 1c) étant réalisée respectivement de manière continue.

7. Système selon l'une quelconque des revendications précédentes, le premier dispositif de réception (4, 4a, 4b) étant prévu dans la région radialement extérieure du premier corps de base (2) et du premier dispositif de serrage (3), et/ou le deuxième dispositif de réception (7, 7a, 7b) étant prévu dans la région radialement extérieure du deuxième corps de base (5) et du deuxième dispositif de serrage (6), le premier dispositif de réception (4, 4a, 4b) étant de préférence adapté pour fixer le premier épaississement (1b) du dispositif d'étanchéité (1), et/ou le deuxième dispositif de réception (7, 7a, 7b) étant adapté pour fixer le deuxième épaississement (1c) du dispositif d'étanchéité (1).

8. Système selon l'une quelconque des revendications 1 à 7, la première rainure (4a), la deuxième rainure (4b), la troisième rainure (7a) et/ou la quatrième rainure (7b) présentant une section transversale essentiellement semi-circulaire, semi-elliptique ou polygonale.

9. Système selon l'une quelconque des revendications précédentes, le premier corps de base (2) et le premier dispositif de serrage (3) pouvant être reliés par un premier dispositif de liaison (8), et/ou le deuxième corps de base (5) et le deuxième dispositif de serrage (6) pouvant être reliés par un deuxième dispositif de liaison (9).

10. Système selon la revendication 9, le premier dispositif de liaison (8) et/ou le deuxième dispositif de liaison (9) étant réalisés sous forme de couple de filetages, de vis, de fermeture à baïonnette ou de clip.

11. Système selon la revendication 9, le premier dispositif de liaison (8) étant une vis avec laquelle le premier corps de base (2) et le premier dispositif de serrage (3) peuvent être reliés l'un à l'autre, et/ou le deuxième dispositif de liaison (9) étant une paire de filetages qui comprend un filetage intérieur (9a) qui est prévu sur le deuxième corps de base (5) et un filetage extérieur (9b) qui est prévu sur le deuxième dispositif de serrage (6).

12. Système selon l'une quelconque des revendications précédentes, le deuxième corps de base (5) et le deuxième dispositif de serrage (6) présentant respectivement un évidement (5b, 6b) dans lequel le premier corps de base (2) est disposé de manière concentrique.

13. Système selon l'une quelconque des revendications précédentes, le deuxième corps de base (5) étant mobile par rapport au premier corps de base (2).

14. Système selon la revendication 13, lequel est un entraînement de trémie de dosage, un entraînement de plateau de distribution, un entraînement de chargement d'une balance à têtes multiples ou sert à l'introduction de force dans un capteur de force.

15. Système selon l'une quelconque des revendications précédentes, le corps principal (1a) présentant une plus grande élasticité que le premier et/ou le deuxième épaississement (1b, 1c).
